# FASCICULE DE BREVET EUROPEEN

(11) **EP 1 870 085 B1**
(45) Date de publication et mention de la délivrance du brevet: **22.07.2015**
(21) Numéro de dépôt: 07109892.5
(22) Date de dépôt: 08.06.2007
(51) Int. Cl.: A61K 8/41, A61K 8/49, A61Q 5/10

(54) **Composition de coloration des fibres kératiniques comprenant la 2,3-diamino-6,7-dihydro-1h,5h-pyrazolo[1,2-a]pyrazol-1-one, la para-phénylènediamine ou la para-toluènediamine et un méta-aminophénol substitué**
Zusammensetzung zur Färbung von Keratinfasern, die 2,3-Diamino-6,7-dihydro-1H,5H-pyrazolo[1,2-a]pyrazol-1-on, para-Phenylendiamin oder para-Toluoldiamin und ein substituiertes meta-Aminophenol enthält
Composition for dyeing keratinous fibres comprising 2,3-diamino-6,7-dihydro-1H,5H-pyrazolo[1,2-a]pyrazol-1-one, para-phenylenediamine or para-toluenediamine and a substituted meta-aminophenol

(30) Priorité: 20.06.2006 FR 0652557
(43) Date de publication de la demande: 26.12.2007
(73) Titulaire: L'Oréal, 75008 Paris (FR)
(72) Inventeur: Cottard, François, 92400, Courbevoie (FR); Desenne, Patricia, 92270, Bois Colombes (FR)
(74) Mandataire: Wattremez, Catherine

(56) Documents cités:
- EP-A- 1 550 656
- DE-A1- 10 148 847

## Description

L'invention a pour objet une composition pour la teinture des fibres kératiniques, et en particulier des fibres kératiniques humaines telles que les cheveux, comprenant la 2,3-diamino-6,7-dihydro-1H,5H-pyrazolo[1,2-a]pyrazol-1-one à titre de première base d'oxydation, la para-phénylènediamine ou la para-toluènediamine à titre de deuxième base d'oxydation et un méta-aminophénol substitué à titre de premier coupleur.

Il est connu de teindre les fibres kératiniques, et en particulier les fibres kératiniques humaines telles que les cheveux, avec des compositions tinctoriales comprenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, d'uniformité de la couleur et de la ténacité aux agents extérieurs.

L'utilisation de ces bases à pH neutre ne permet pas d'atteindre une gamme de nuances variées, en particulier pour les nuances chaudes telles que les rouges et les orangés.

La demande de brevet Européen EP1550656 décrit des compositions tinctoriales des cheveux comprenant la 2,3-diamino-6,7-dihydro-1H,5H-pyrazolo[1,2-a]pyrazol-1-one comme base d'oxydation et l'un des coupleurs 6-chloro-2-méthyl-5-amino-phénol, 2-(2,4-diamino-phénoxy)-éthanol ou 5-amino-2-méthyl-phénol. Les nuances observées sont orangé ou rouge chromatique.

Le but de la présente invention est de fournir de nouvelles compositions de coloration des fibres kératiniques qui permettent d'obtenir une coloration aux nuances rouges profonds particulièrement visibles, puissante, chromatique, esthétique, peu sélective et résistant bien aux diverses agressions que peuvent subir les cheveux tels que les shampooings, la lumière, la sueur et les déformations permanentes.

La présente invention a donc pour objet une composition de coloration des fibres kératiniques comprenant, dans un milieu approprié :
- au moins une première base d'oxydation choisie parmi la 2,3-diamino-6,7-dihydro-1H,5H-pyrazolo[1,2-a]pyrazol-1-one de formule (I) suivante et ses sels d'addition : 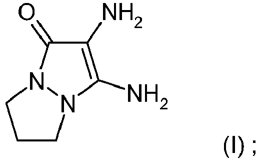
- au moins une deuxième base d'oxydation choisie parmi la para-phénylènediamine, la para-toluènediamine et leurs sels d'addition ; et
- au moins un premier coupleur choisi parmi les méta-aminophénols substitués de formule (II) suivante et leurs sels d'addition : 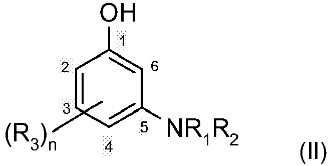 dans laquelle :
   R₁ et R₂, identiques ou différents, représentent un atome d'hydrogène ; un radical alkyle ; un radical monohydroxyalkyle ; un radical polyhydroxyalkyle ; un radical monoaminoalkyle ; ou
   R₁ et R₂ forment entre eux et avec l'atome d'azote auquel ils sont reliés un groupement cyclique contenant un ou plusieurs hétéroatomes, de 5 à 7 chaînons, saturé ou insaturé, non substitué ou substitué par un ou plusieurs radicaux choisis parmi les radicaux carboxy, carboxamido, hydroxyle, amino, mono ou dialkylamino, alkyle éventuellement substitué par un ou plusieurs radicaux hydroxyle, amino, mono ou dialkylamino ;
   R₃ représentent, indépendamment les uns des autres, un atome d'halogène ; un radical alkyle ; un radical alcoxy ; un radical monohydroxyalkyle ; un radical polyhydroxyalkyle ; un radical monohydroxyalcoxy ; un radical polyhydroxyalcoxy ;
   n est un entier compris entre 0 et 4 ;
   sous réserve que lorsque n est égal à 0, alors au moins l'un des radicaux R₁ et R₂ est différent d'un atome d'hydrogène ;
   étant entendu que le ratio molaire premier coupleur / première base d'oxydation est supérieur à 1, le ratio molaire première base d'oxydation / deuxième base d'oxydation est compris entre 0,5 et 1,5 et la quantité molaire de la première base d'oxydation est supérieure ou égale à 2,5.10⁻³ mole pour 100 g de composition.

La présente invention permet d'obtenir une coloration des fibres kératiniques aux nuances rouges profonds particulièrement visibles, en particulier une coloration sur cheveux gris à 90 % de blancs naturels ou permanentés présentant selon la notation CIELAB une valeur de L* inférieure ou égale à 50, une valeur de a* comprise entre 10 et 25, une valeur de b* comprise entre 3 et 25 et un rapport b* / a* compris entre 0,3 et 1, de préférence entre 0,3 et 0,7.

La présente invention permet également d'obtenir une coloration puissante, esthétique, particulièrement peu sélective et résistant bien aux diverses agressions que peuvent subir les cheveux tels que les shampooings, la lumière, la sueur et les déformations permanentes. Elle permet de plus d'obtenir une coloration intense à pH neutre.

Un autre objet de l'invention est un procédé de teinture des fibres kératiniques mettant en oeuvre la composition de la présente invention, ainsi que l'utilisation de cette composition pour la teinture des fibres kératiniques.

L'invention a enfin pour objet un kit de coloration comprenant d'une part une composition de coloration contenant la 2,3-diamino-6,7-dihydro-1H,5H-pyrazolo[1,2-a]pyrazol-1-one à titre de première base d'oxydation, la para-phénylènediamine ou la para-toluènediamine à titre de deuxième base d'oxydation et un méta-aminophénol substitué à titre de coupleur et d'autre part une composition contenant un agent oxydant.

La notation CIELAB utilisée dans le cadre de l'invention définit un espace colorimétrique dans lequel chaque couleur est définie par 3 paramètres (L*, a*, b*). Le paramètre L* reflète la clarté de la couleur , la valeur L* étant égale à 0 pour le noir et égale à 100 pour le blanc absolu. Plus la valeur de L* est élevée, moins la coloration est intense. Le paramètre a* correspond à l'axe du couple antagoniste vert / rouge. Le paramètre b* correspond à l'axe du couple antagoniste bleu / jaune.

A moins d'une indication différente, les bornes des gammes de valeurs qui sont données dans le cadre de la présente invention sont incluses dans ces gammes.

Dans le cadre de l'invention, on entend par radical alkyle des radicaux alkyle linéaires ou ramifiés en C₁-C₁₀ sauf indication contraire, préférentiellement en C₁-C₆, encore plus préférentiellement en C₁-C₄, tels que le radical méthyle, éthyle, propyle, isopropyle, isobutyle, tertiobutyle, pentyle, hexyle.

Dans le cadre de la présente invention, le ou les hétéroatomes peuvent être choisis parmi un atome d'oxygène, un atome d'azote, un atome de soufre, un atome de phosphore.

Dans le cadre de la présente invention, un atome d'halogène peut être choisi parmi un atome de chlore, un atome de brome, un atome d'iode et un atome de fluor.

Selon un mode de réalisation particulier de l'invention, R₁ et R₂ représentent, indépendamment l'un de l'autre, un atome d'hydrogène ; un radical alkyle par exemple un radical méthyle ou éthyle ; un radical monohydroxyalkyle par exemple un radical β-hydroxyéthyle ou γ-hydroxypropyle ; ou R₁ et R₂ forment entre eux et avec l'atome d'azote auquel ils sont reliés un cycle choisi parmi les hétérocycles pyrrolidine, pipéridine, homopipéridine, pipérazine, homopipérazine, morpholine ; lesdits cycles pouvant être substitués par un ou plusieurs radicaux hydroxyle, amino, mono ou dialkyl(C₁-C₂)amino, carboxy, carboxamido, alkyle en C₁-C₄ éventuellement substitué par un ou plusieurs radicaux hydroxyle, amino, mono ou dialkyl(C₁-C₂)amino, et plus particulièrement choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(β-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, la 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine, la pipérazine, la 4-méthyl-pipérazine, la 4-éthyl-pipérazine, la 4-(β-hydroxyéthyl)-pipérazine, la morpholine, et plus particulièrement ils forment un groupement pyrrolidin-1-yle ; pipéridin-1-yle ; pipérazin-1-yle ; 4-méthyl-pipérazin-1-yle ; 4-éthyl-pipérazin-1-yle ; 4-(β-hydroxyéthyl)-pipérazin-1-yle ; morpholin-4-yle.

Selon un mode de réalisation particulier de l'invention, R₃ est choisi parmi un atome d'halogène, un radical alkyle, un radical alcoxy, un radical monohydroxyalcoxy. A titre d'exemple, R₃ est choisi parmi un atome de chlore, un radical méthyle, un radical méthoxy, un radical β-hydroxyéthyloxy.

Selon un mode de réalisation particulier de l'invention, n est compris entre 0 et 2. A titre d'exemple, n est égal à 1 ou 2. Lorsque n est égal à 1, R₃ peut se situer en position 2 et lorsque n est égal à 2, R₃ peuvent se situer en positions 2 et 4 ou en position 2 et 6.

Parmi les méta-aminophénols substitués de formule (II) utiles dans le cadre de l'invention, on peut plus particulièrement citer le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 6-chloro 2-méthyl 5-amino phénol, le 5-amino 2,4-diméthoxy phénol, le 5-(γ-hydroxypropylamino) 2-méthyl phénol, le 3-diméthylamino-phénol ; le 2-méthyl-5-diméthylamino-phénol ; le 2-éthyl-5-diméthylamino-phénol ; le 2-méthoxy-5-diméthylamino-phénol ; le 2-éthoxy-5-diméthylamino-phénol ; le 2-(β-hydroxyéthyl)-5-diméthylamino-phénol ; le 3-diéthylamino-phénol ; le 2-méthyl-5-diéthylamino-phénol ; le 2-éthyl-5-diéthylamino-phénol ; le 2-méthoxy-5-diéthylamino-phénol ; le 2-éthoxy-5-diéthylamino-phénol ; le 2-(β-hydroxyéthyi)-5-diéthyiamino-phénol ; le 3-di(β-hydroxyéthyl)amino-phénol ; le 2-méthyl-5-di(β-hydroxyéthyl)amino-phénol ; le 2-éthyl-5-di(β-hydroxyéthyl)amino-phénol ; le 2-méthoxy-5-di(β-hydroxyéthyl)amino-phénol ; le 2-éthoxy-5-di(β-hydroxyéthyl)amino-phénol ; le 2-(β-hydroxyéthyl)-5-di(β-hydroxyéthyl)amino-phénol ; le 3-pyrrolidin-1-yl-phénol ; le 2-méthyl-5-pyrrolidin-1-yl-phénol ; le 2-éthyl-5-pyrrolidin-1-yl-phénol ; le 2-méthoxy-5-pyrrolidin-1-yl-phénol ; le 2-éthoxy-5-pyrrolidin-1-yl-phénol ; le 2-(β-hydroxyéthyl)-5-pyrrolidin-1-yl-phénol ; le 3-pipéridin-1-yl-phénol ; le 2-méthyl-5-pipéridin-1-yl-phénol ; le 2-éthyl-5-pipéridin-1-yl-phénol ; le 2-méthoxy-5-pipéridin-1-yl-phénol ; le 2-éthoxy-5-pipéridin-1-yl-phénol ; le 2-(β-hydroxyéthyl)-5-pipéridin-1-yl-phénol ; le 3-pipérazin-1-yl-phénol ; le 2-méthyl-5-pipérazin-1-yl-phénol ; le 2-éthyl-5-pipérazin-1-yl-phénol ; le 2-méthoxy-5-pipérazin-1-yl-phénol ; le 2-éthoxy-5-pipérazin-1-yl-phénol ; le 2-(β-hydroxyéthyl)-5-pipérazin-1-yl-phénol ; le 3-(4-méthyl-pipérazin-1-yl)-phénol ; le 2-méthyl-5-(4-méthyl-pipérazin-1-yl)-phénol ; le 2-éthyl-5-(4-méthyl-pipérazin-1-yl)-phénol ; le 2-méthoxy-5-(4-méthyl-pipérazin-1-yl)-phénol ; le 2-éthoxy-5-(4-méthyl-pipérazin-1-yl)-phénol ; le 2-(β-hydroxyéthyl)-5-(4-méthyl-pipérazin-1-yl)-phénol ; le 3-(4-éthyl-pipérazin-1-yl)-phénol ; le 2-méthyl-5-(4-éthyl-pipérazin-1-yl)-phénol ; le 2-éthyl-5-(4-éthyl-pipérazin-1-yl)-phénol ; le 2-méthoxy-5-(4-éthyl-pipérazin-1-yl)-phénol ; le 2-éthoxy-5-(4-éthyl-pipérazin-1-yl)-phénol ; le 2-(β-hydroxyéthyl)-5-(4-éthyl-pipérazin-1-yl)-phénol ; le 3-(4-(β-hydroxyéthyl)-pipérazin-1-yl)-phénol ; le 2-méthyl-5-(4-((3-hydroxyéthyl)-pipérazin-1-yl)-phénol ; le 2-éthyl-5-(4-(β-hydroxyéthyl)-pipérazin-1-yl)-phénol ; le 2-méthoxy-5-(4-(β-hydroxyéthyl)-pipérazin-1-yl)-phénol ; le 2-éthoxy-5-(4-(β-hydroxyéthyl)-pipérazin-1-yl)-phénol ; le 2-(β-hydroxyéthyl)-5-(4-(β-hydroxyéthyl)-pipérazin-1-yl)-phénol ; le 3-morpholin-4-yl-phénol ; le 2-méthyl-5-morpholin-4-yl-phénol ; le 2-éthyl-5-morpholin-4-yl-phénol ; le 2-méthoxy-5-morpholin-4-yl-phénol ; le 2-éthoxy-5-morpholin-4-yl-phénol ; le 2-(β-hydroxyéthyl)-5-morpholin-4-yl-phénol.

Parmi les méta-aminophénols substitués de formule (II) cités ci-dessus, le 5-N-(β-hydroxyéthylamino) 2-méthyl phénol, le 5-amino 2-méthyl phénol et le 6-chloro 2-méthyl 5-amino phénol sont particulièrement préférés.

Dans la composition conforme à la présente invention, le ratio molaire premier coupleur / première base d'oxydation est supérieur à 1. De préférence, le ratio molaire premier coupleur / première base d'oxydation est compris entre 2 et 5.

Dans la composition conforme à la présente invention, le ratio molaire première base d'oxydation / deuxième base d'oxydation est compris entre 0,5 et 1,5. De préférence, le ratio molaire première base d'oxydation / deuxième base d'oxydation est compris entre 0,7 et 1,3.

La composition tinctoriale de l'invention peut contenir d'autres bases d'oxydation différentes de celles utiles dans la présente invention et conventionnellement utilisées pour la teinture de fibres kératiniques.

La composition de la présente invention peut par exemple comprendre au moins une base d'oxydation additionnelle choisie parmi les para-phénylènediamines autres que la para-phénylènediamine, la para-toluènediamine et leurs sels d'addition, les bis-phénylalkylènediamines, les para-aminophénols, les bis-para-aminophénols, les ortho-aminophénols, les ortho-phénylènediamines, les bases hétérocycliques différentes de la 2,3-diamino-6,7-dihydro-1H,5H-pyrazolo[1,2-a]pyrazol-1-one et ses sels d'addition.

Parmi les para-phénylènediamines, on peut citer, à titre d'exemple, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition.

Parmi les para-phénylènediamines citées ci-dessus, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition sont particulièrement préférées.

Parmi les bis-phénylalkylènediamines, on peut citer, à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols utilisables à titre de bases d'oxydation dans les compositions tinctoriales conformes à l'invention, on peut notamment citer les composés répondant à la formule (III) suivante, et leurs sels d'addition : 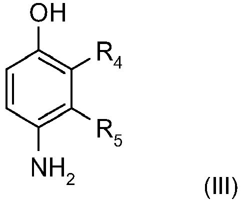 dans laquelle :
R₄ représente un atome d'hydrogène ; un atome d'halogène ; un radical alkyle ; un radical monohydroxyalkyle ; un radical alcoxyalkyle ; un radical aminoalkyle ; un radical hydroxyalkylaminoalkyle ;
R₅ représente un atome d'hydrogène ; un atome d'halogène ; un radical alkyle ; un radical monohydroxyalkyle ; un radical polyhydroxyalkyle ; un radical aminoalkyle ; un radical cyanoalkyle ; un radical alcoxyalkyle ;
étant entendu qu'au moins un des radicaux R₄ ou R₅ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition.

Parmi les ortho-aminophénols, on peut citer, à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer, à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; le 3-amino-pyrazolo[1,5-a]pyridine-5-ol ; le 3-amino-pyrazolo[1,5-a]pyridine-4-ol ; le 3-amino-pyrazolo[1,5-a]pyridine-6-ol ; le 3-amino-pyrazolo[1,5-a]pyridine-7-ol ; ainsi que leurs d'addition.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique.

Parmi les dérivés pyrazoliques, on peut citer les composés décrits dans les brevets DE 38 43 892, DE 41 33 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

De préférence, la composition conforme à l'invention comprend au moins une base d'oxydation additionnelle choisie parmi les para-aminophénols. Encore plus préférentiellement, la composition conforme à l'invention comprend au moins une base d'oxydation additionnelle choisie parmi le para-aminophénol et ses sels d'addition.

La composition tinctoriale de l'invention peut contenir des coupleurs additionnels différents de ceux utiles dans la présente invention et conventionnellement utilisés pour la teinture de fibres kératiniques.

La composition de la présente invention peut par exemple comprendre au moins un coupleur additionnel choisi parmi les méta-phénylènediamines, les méta-aminophénols différents des méta-aminophénols de formule (II) et leurs sels d'addition, les méta-diphénols, les coupleurs naphtaléniques, les coupleurs hétérocycliques.

A titre d'exemples, on peut citer le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène et leurs sels d'addition.

De préférence, la composition conforme à l'invention comprend au moins un coupleur additionnel choisi parmi la 2-amino 3-hydroxy pyridine, le 1,3-dihydroxy 2-méthyl benzène et leurs sels d'addition.

La ou les bases d'oxydation présentes dans la composition de l'invention sont en général présentes chacune en quantité comprise entre 0,001 à 10 % en poids environ du poids total de la composition tinctoriale, de préférence entre 0,005 et 6 %.

Le ou les coupleurs présents dans la composition de l'invention sont en général présents chacun en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les alkyl(C₁-C₄)sulfonates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymériques anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 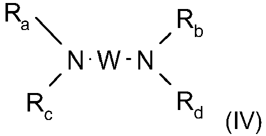 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. De préférence, cette coloration est révélée à pH neutre.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus à l'exception de l'agent oxydant et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la coloration d'oxydation des fibres kératiniques, et en particulier des fibres kératiniques humaines telles que les cheveux, d'une composition telle que définie précédemment.

Selon un mode de réalisation particulier, l'utilisation de la composition conforme à l'invention sur cheveux gris à 90 % de blancs naturels ou permanentés permet d'obtenir une coloration présentant selon la notation CIELAB une valeur de L* inférieure ou égale à 50, une valeur de a* comprise entre 10 et 25, une valeur de b* comprise entre 3 et 25 et un rapport b* / a* compris entre 0,3 et 1, de préférence entre 0,3 et 0,7.

Les exemples qui suivent servent à illustrer l'invention sans toutefois présenter un caractère limitatif.

### EXEMPLES

### Exemple 1 :

La composition 1 suivante a été réalisée :

| | |
|---|---|
| Acide diéthylène triamine pentacétique, sel pentasodique en solution aqueuse à 40 % | 2 g |
| Ammoniac en solution aqueuse à 20 % | 12 g |
| Métabisulfite de sodium en poudre | 0,71 g |
| Monoéthanolamine pure | 1,35 g |
| Silice pyrogénée à caractère hydrophobe | 1,2 g |
| 2,3-diamino-6,7-dihydro-1H,5H-pyrazolo[1,2-a]pyrazol-1-one, 2 CH₃-SO₃H | 1,87 g |
| 5-amino 2-méthyl phénol | 1,96 g |
| Oxyde de titane (anatase non traité) enrobé de polydiméthylsiloxane (98/2) | 0,15 g |
| Para-phénylènediamine | 0,77 g |
| Distéarate de glycol | 2 g |
| Mica-Oxyde de titane-Oxyde de fer brun (58/37,5/4,5) | 0,5 g |
| Parfum | 0,5 g |
| Polycondensat tétraméthyl hexaméthylènediamine / dichloro 1,3-propylène en solution aqueuse | 3 g |
| Chlorure de poly diméthyl diallyl ammonium dans l'eau à 40 % non stabilisé | 5 g |
| Polymère carboxyvinylique synthétisé dans le mélange acétate d'éthyle / cyclohexane | 0,6 g |
| Eau désionisée | 23,64 g |
| Propylène glycol | 7 g |
| Acide laurique naturel | 3 g |
| Alcool laurique oxyéthyléné (12 OE) | 7 g |
| Alcool décylique oxyéthyléné (3 OE) | 10 g |
| Alcool cétylstéarylique (C16-18 50/50) (origine synthétique) | 11,5 g |
| Alcool oléocétylique oxyéthyléné (30 OE) | 4 g |
| Vitamine C : acide L-ascorbique en poudre fine | 0,25 g |

Au moment de l'emploi, 1 partie en poids de la composition 1 est mélangée avec 1,5 parties en poids d'une solution de peroxyde d'hydrogène à 25 volumes à pH 2,2. On obtient un pH final de 9,6.

Le mélange obtenu est appliqué sur des mèches de cheveux gris à 90 % de blancs naturels ou permanentés. Après 20 minutes de pose à température ambiante, les mèches sont rincées, lavées avec un shampooing standard, rincées à nouveau puis séchées.

La coloration capillaire est évaluée de manière visuelle. On obtient une nuance châtain rouge.

La couleur des cheveux est mesurée à l'aide d'un spectrocolorimètre MINOLTA CM2002® (illuminant D65 - 10 ° CSI) dans le système CIELab.

Les résultats obtenus sont présentés dans le tableau ci-dessus.

| **Type de cheveux** | **L*** | **a*** | **b*** |
|---|---|---|---|
| Blancs naturels | 25,9 | 15,7 | 6,4 |
| Blancs permanentés | 23,2 | 12,6 | 5,1 |

### Exemple 2 :

La composition 2 suivante a été réalisée :

| | |
|---|---|
| Acide diéthylène triamine pentacétique, sel pentasodique en solution aqueuse à 40 % | 2 g |
| Ammoniac en solution aqueuse à 20 % | 12 g |
| Métabisulfite de sodium en poudre | 0,71 g |
| Monoéthanolamine pure | 1,35 g |
| Silice pyrogénée à caractère hydrophobe | 1,2 g |
| 2,3-diamino-6,7-dihydro-1H,5H-pyrazolo[1,2-a]pyrazol-1-one, 2 CH₃-SO₃H | 1,87 g |
| 5-amino 2-méthyl phénol | 1,96 g |
| Oxyde de titane (anatase non traité) enrobé de polydiméthylsiloxane (98/2) | 0,15 g |
| Para-phénylènediamine | 0,77 g |
| Para-aminophénol | 0,15 g |
| Distéarate de glycol | 2 g |
| Mica-Oxyde de titane-Oxyde de fer brun (58/37,5/4,5) | 0,5 g |
| Parfum | 0,5 g |
| Polycondensat tétraméthyl hexaméthylènediamine / dichloro 1,3-propylène en solution aqueuse | 3 g |
| Chlorure de poly diméthyl diallyl ammonium dans l'eau à 40 % non stabilisé | 5 g |
| Polymère carboxyvinylique synthétisé dans le mélange acétate d'éthyle / cyclohexane | 0,6 g |
| Eau désionisée | 23,49 g |
| Propylène glycol | 7 g |
| Acide laurique naturel | 3 g |
| Alcool laurique oxyéthyléné (12 OE) | 7 g |
| Alcool décylique oxyéthyléné (3 OE) | 10 g |
| Alcool cétylstéarylique (C16-18 50/50) (origine synthétique) | 11,5 g |
| Alcool oléocétylique oxyéthyléné (30 OE) | 4 g |
| Vitamine C : acide L-ascorbique en poudre fine | 0,25 g |

Le mode opératoire de teinture est le même que celui utilisé dans l'exemple 1.

La coloration capillaire est évaluée de manière visuelle. On obtient une nuance châtain clair rouge.

La couleur des cheveux est mesurée à l'aide d'un spectrocolorimètre MINOLTA CM2002® (illuminant D65 - 10 ° CSI) dans le système CIELab.

Les résultats obtenus sont présentés dans le tableau ci-dessus.

| **Type de cheveux** | **L*** | **a*** | **b*** |
|---|---|---|---|
| Blancs naturels | 25,7 | 15,6 | 7,4 |
| Blancs permanentés | 23,9 | 14,6 | 6,4 |

## Revendications

1. Composition de coloration des fibres kératiniques comprenant, dans un milieu approprié :
• au moins une première base d'oxydation choisie parmi la 2,3-diamino-6,7-dihydro-1 H,5H-pyrazolo[1,2-a]pyrazol-1-one et ses sels d'addition ;
• au moins une deuxième base d'oxydation choisie parmi la para-phénylènediamine, la para-toluènediamine et leurs sels d'addition ; et
• au moins un premier coupleur choisi parmi les méta-aminophénols substitués de formule (II) suivante et leurs sels d'addition : 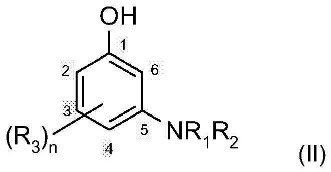 dans laquelle :
R₁ et R₂, identiques ou différents, représentent un atome d'hydrogène ; un radical alkyle ; un radical monohydroxyalkyle ; un radical polyhydroxyalkyle ; un radical monoaminoalkyle ; ou
R₁ et R₂ forment entre eux et avec l'atome d'azote auquel ils sont reliés un groupement cyclique contenant un ou plusieurs hétéroatomes, de 5 à 7 chaînons, saturé ou insaturé, non substitué ou substitué par un ou plusieurs radicaux choisis parmi les radicaux carboxy, carboxamido, hydroxyle, amino, mono ou dialkylamino, alkyle éventuellement substitué par un ou plusieurs radicaux hydroxyle, amino, mono ou dialkylamino ;
R₃ représentent, indépendamment les uns des autres, un atome d'halogène ; un radical alkyle ; un radical alcoxy ; un radical monohydroxyalkyle ; un radical polyhydroxyalkyle ; un radical monohydroxyalcoxy ; un radical polyhydroxyalcoxy ;
n est un entier compris entre 0 et 4 ;
sous réserve que lorsque n est égal à 0, alors au moins l'un des radicaux R₁ et R₂ est différent d'un atome d'hydrogène ;
étant entendu que le ratio molaire premier coupleur / première base d'oxydation est supérieur à 1, le ratio molaire première base d'oxydation / deuxième base d'oxydation est compris entre 0,5 et 1,5 et la quantité molaire de la première base d'oxydation est supérieure ou égale à 2,5.10⁻³ mole pour 100 g de composition.

2. Composition selon la revendication 1 dans laquelle R₁ et R₂ représentent, indépendamment l'un de l'autre, un atome d'hydrogène ; un radical alkyle ; un radical monohydroxyalkyle ; ou R₁ et R₂ forment entre eux et avec l'atome d'azote auquel ils sont reliés un cycle choisi parmi les hétérocycles pyrrolidine, pipéridine, homopipéridine, pipérazine, homopipérazine, morpholine ; lesdits cycles pouvant être substitués par un ou plusieurs radicaux hydroxyle, amino, mono ou dialkyl(C₁-C₂)amino, carboxy, carboxamido, alkyle en C₁-C₄ éventuellement substitué par un ou plusieurs radicaux hydroxyle, amino, mono ou dialkyl(C₁-C₂)amino.

3. Composition selon la revendication 1 ou 2 dans laquelle R₃ est choisi parmi un atome d'halogène, un radical alkyle, un radical alcoxy, un radical monohydroxyalcoxy.

4. Composition selon l'une quelconque des revendications précédentes dans laquelle n est compris entre 0 et 2.

5. Composition selon l'une quelconque des revendications précédentes dans laquelle le ou les méta-aminophénols substitués de formule (II) sont choisis parmi le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 6-chloro 2-méthyl 5-amino phénol, le 5-amino 2,4-diméthoxy phénol, le 5-(γ-hydroxypropylamino) 2-méthyl phénol, le 3-diméthylamino-phénol ; le 2-méthyl-5-diméthylamino-phénol ; le 2-éthyl-5-diméthylamino-phénol ;le 2-méthoxy-5-diméthylamino-phénol ; le 2-éthoxy-5-diméthylamino-phénol ; le 2-(β-hydroxyéthyl)-5-diméthylamino-phénol ; le 3-diéthylamino-phénol ; le 2-méthyl-5-diéthylamino-phénol ; le 2-éthyl-5-diéthylamino-phénol ; le 2-méthoxy-5-diéthylamino-phénol ; le 2-éthoxy-5-diéthylamino-phénol ; le 2-(β-hydroxyéthyi)-5-diéthyiamino-phénoi ; le 3-di(β-hydroxyéthyl)amino-phénol ; le 2-méthyl-5-di(β-hydroxyéthyl)amino-phénol ; le 2-éthyl-5-di(β-hydroxyéthyl)amino-phénol ; le 2-méthoxy-5-di(β-hydroxyéthyl)amino-phénol ; le 2-éthoxy-5-di(β-hydroxyéthyl)amino-phénol ; le 2-(β-hydroxyéthyl)-5-di(β-hydroxyéthyl)amino-phénol ; le 3-pyrrolidin-1-yl-phénol ; le 2-méthyl-5-pyrrolidin-1-yl-phénol ; le 2-éthyl-5-pyrrolidin-1-yl-phénol ; le 2-méthoxy-5-pyrrolidin-1-yl-phénol ; le 2-éthoxy-5-pyrrolidin-1-yl-phénol ; le 2-(β-hydroxyéthyl)-5-pyrrolidin-1-yl-phénol ; le 3-pipéridin-1-yl-phénol ; le 2-méthyl-5-pipéridin-1-yl-phénol ; le 2-éthyl-5-pipéridin-1-yl-phénol ; le 2-méthoxy-5-pipéridin-1-yl-phénol ; le 2-éthoxy-5-pipéridin-1-yl-phénol ; le 2-(β-hydroxyéthyl)-5-pipéridin-1-yl-phénol ; le 3-pipérazin-1-yl-phénol ; le 2-méthyl-5-pipérazin-1-yl-phénol ; le 2-éthyl-5-pipérazin-1-yl-phénol ; le 2-méthoxy-5-pipérazin-1-yl-phénol ; le 2-éthoxy-5-pipérazin-1-yl-phénol ; le 2-(β-hydroxyéthyl)-5-pipérazin-1-yl-phénol ; le 3-(4-méthyl-pipérazin-1-yl)-phénol ; le 2-méthyl-5-(4-méthyl-pipérazin-1-yl)-phénol ; le 2-éthyl-5-(4-méthyl-pipérazin-1-yl)-phénol ; le 2-méthoxy-5-(4-méthyl-pipérazin-1-yl)-phénol ; le 2-éthoxy-5-(4-méthyl-pipérazin-1-yl)-phénol ; le 2-((3-hydroxyéthyl)-5-(4-méthyl-pipérazin-1-yl)-phénol ; le 3-(4-éthyl-pipérazin-1-yl)-phénol ; le 2-méthyl-5-(4-éthyl-pipérazin-1-yl)-phénol ; le 2-éthyl-5-(4-éthyl-pipérazin-1-yl)-phénol ; le 2-méthoxy-5-(4-éthyl-pipérazin-1-yl)-phénol ; le 2-éthoxy-5-(4-éthyl-pipérazin-1-yl)-phénol ; le 2-(β-hydroxyéthyl)-5-(4-éthyl-pipérazin-1-yl)-phénol ; le 3-(4-(β-hydroxyéthyl)-pipérazin-1-yl)-phénol ; le 2-méthyl-5-(4-((3-hydroxyéthyl)-pipérazin-1-yl)-phénol ; le 2-éthyl-5-(4-(β-hydroxyéthyl)-pipérazin-1-yl)-phénol ; le 2-méthoxy-5-(4-(β-hydroxyéthyl)-pipérazin-1-yl)-phénol ; le 2-éthoxy-5-(4-(β-hydroxyéthyl)-pipérazin-1-yl)-phénol ; le 2-(β-hydroxyéthyl)-5-(4-(β-hydroxyéthyl)-pipérazin-1-yl)-phénol ; le 3-morpholin-4-yl-phénol ; le 2-méthyl-5-morpholin-4-yl-phénol ; le 2-éthyl-5-morpholin-4-yl-phénol ; le 2-méthoxy-5-morpholin-4-yl-phénol ; le 2-éthoxy-5-morpholin-4-yl-phénol ; le 2-(β-hydroxyéthyl)-5-morpholin-4-yl-phénol.

6. Composition selon la revendication 5 dans laquelle le ou les méta-aminophénols substitués de formule (II) sont choisis parmi le 5-N-(β-hydroxyéthylamino) 2-méthyl phénol, le 5-amino 2-méthyl phénol et le 6-chloro 2-méthyl 5-amino phénol.

7. Composition selon l'une quelconque des revendications précédentes dans laquelle le ratio molaire premier coupleur / première base d'oxydation est compris entre 2 et 5.

8. Composition selon l'une quelconque des revendications précédentes dans laquelle le ratio molaire première base d'oxydation / deuxième base d'oxydation est compris entre 0,7 et 1,3.

9. Composition selon l'une quelconque des revendications précédentes comprenant au moins une base d'oxydation additionnelle choisie parmi les para-aminophénols.

10. Composition selon la revendication 9 comprenant au moins une base d'oxydation additionnelle choisie parmi le para-aminophénol et ses sels d'addition.

11. Composition selon l'une quelconque des revendications précédentes comprenant au moins un coupleur additionnel choisi parmi la 2-amino 3-hydroxy pyridine, le 1,3-dihydroxy 2-méthyl benzène et leurs sels d'addition.

12. Composition selon l'une quelconque des revendications précédentes comprenant de plus un agent oxydant.

13. Procédé de teinture des fibres kératiniques, **caractérisé en ce qu'**une composition telle que définie dans l'une quelconque des revendications 1 à 11 est appliquée sur les fibres kératiniques en présence d'un agent oxydant pendant un temps suffisant pour développer la coloration désirée.

14. Dispositif à plusieurs compartiments dans lequel un premier compartiment contient une composition tinctoriale telle que définie dans l'une quelconque des revendications 1 à 11 et un deuxième compartiment contient un agent oxydant.

15. Utilisation pour la teinture d'oxydation des fibres kératiniques d'une composition telle que définie dans l'une quelconque des revendications 1 à 12.

16. Utilisation selon la revendication 15 pour obtenir une coloration sur cheveux gris à 90 % de blancs naturels ou permanentés présentant selon la notation CIELAB une valeur de L* inférieure ou égale à 50, une valeur de a* comprise entre 10 et 25, une valeur de b* comprise entre 3 et 25 et un rapport b* / a* compris entre 0,3 et 1.

## Patentansprüche

1. Zusammensetzung zum Färben von Keratinfasern, umfassend in einem geeigneten Medium:
• mindestens eine erste Oxidationsbase, die aus 2,3-Diamino-6,7-dihydro-1H,5H-pyrazolo[1,2-a]-pyrazol-1-on und Additionssalzen davon ausgewählt ist;
• mindestens eine zweite Oxidationsbase, die aus para-Phenylendiamin, para-Toluoldiamin und Additionssalzen davon ausgewählt ist; und
• mindestens einen ersten Kuppler, der aus substituierten meta-Aminophenolen der folgenden Formel (II) und Additionssalzen davon ausgewählt ist: 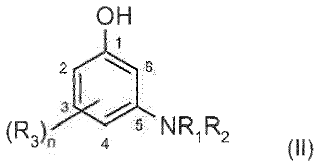 worin:
R₁ und R₂ gleich oder verschieden sind und für ein Wasserstoffatom, einen Alkylrest, einen Monohydroxyalkylrest, einen Polyhydroxyalkylrest oder einen Monoaminoalkylrest stehen;
R₁ und R₂ miteinander und mit dem Stickstoffatom, an das sie gebunden sind, eine gesättigte oder ungesättigte, 5- bis 7-gliedrige cyclische Gruppe, die ein oder mehrere Heteroatome enthält und gegebenenfalls durch einen oder mehrere aus Carboxy-, Carboxamido-, Hydroxyl-, Amino-, Mono- oder Dialkylaminoresten und gegebenenfalls durch einen oder mehrere Hydroxyl-, Amino-, Mono- oder Dialkylaminoreste substituierten Alkylresten ausgewählte Reste substituiert ist, bilden;
R₃ unabhängig voneinander für ein Halogenatom, einen Alkylrest, einen Alkoxyrest, einen Monohydroxyalkylrest, einen Polyhydroxyalkylrest, einen Monohydroxyalkoxyrest oder einen Polyhydroxyalkoxyrest stehen und
n für eine ganze Zahl zwischen 0 und 4 steht:
mit der Maßgabe, dass dann, wenn n gleich 0 ist, mindestens einer der Reste R₁ und R₂ von einem Wasserstoffatom verschieden ist;
wobei es sich versteht, dass das Molverhältnis von erstem Kuppler zu erster Oxidationsbase größer als 1 ist, das Molverhältnis von erster Oxidationsbase zu zweiter Oxidationsbase zwischen 0,5 und 1,5 liegt und die molare Menge der ersten Oxidationsbase größer oder gleich 2,5.10⁻³ mol pro 100 g Zusammensetzung ist.

2. Zusammensetzung nach Anspruch 1, in der R₁ und R₂ unabhängig voneinander für ein Wasserstoffatom, einen Alkylrest oder einen Monohydroxyalkylrest stehen oder R₁ und R₂ miteinander und mit dem Stickstoffatom, an das sie gebunden sind, einen Ring bilden, der aus Pyrrolidin-, Piperidin-, Homopiperidin-, Piperazin-, Homopiperazin- und Morpholin-Heterocyclen ausgewählt ist, wobei die Ringe durch einen oder mehrere aus Hydroxyl-, Amino-, Mono- oder Di(C₁-C₂)alkylamino-, Carboxy- und Carboxamidoresten und gegebenenfalls durch einen oder mehrere Hydroxyl-, Amino-, Mono- oder Di(C₁-C₂)alkylaminoreste substituierten C₁-C₄-Alkylresten ausgewählte Reste substituiert sein können.

3. Zusammensetzung nach Anspruch 1 oder 2, in der R₃ aus einem Halogenatom, einem Alkylrest, einem Alkoxyrest und einem Monohydroxyalkoxyrest ausgewählt ist.

4. Zusammensetzung nach einem der vorhergehenden Ansprüche, in der n zwischen 0 und 2 liegt.

5. Zusammensetzung nach einem der vorhergehenden Ansprüche, in der das bzw. die substituierten meta-Aminophenole der Formel (II) aus 5-Amino-2-methoxyphenol, 5-Amino-2-(β-hydroxyethyloxy)-phenol, 5-Amino-2-methylphenol, 5-N-(β-Hydroxyethyl)amino-2-methylphenol, 5-N-(β-Hydroxyethyl)amino-4-methoxy-2-methylphenol, 5-Amino-4-methoxy-2-methylphenol, 5-Amino-4-chlor-2-methylphenol, 6-Chlor-2-methyl-5-aminophenol, 5-Amino-2,4-dimethoxyphenol, 5-(γ-Hydroxypropyl-amino)-2-methylphenol, 3-Dimethylaminophenol, 2-Methyl-5-dimethylaminophenol, 2-Ethyl-5-dimethylaminophenol, 2-Methoxy-5-dimethylaminophenol, 2-Ethoxy-5-dimethylaminophenol, 2-(β-Hydroxyethyl)-5-dimethylaminophenol, 3-Diethylaminophenol, 2-Methyl-5-diethylaminophenol, 2-Ethyl-5-diethylaminophenol, 2-Methoxy-5-diethylaminophenol, 2-Ethoxy-5-diethylaminophenol, 2-(β-Hydroxyethyl)-5-diethylaminophenol, 3-Di(β-hydroxyethyl)amino-phenol, 2-Methyl-5-di(β-hydroxyethyl)aminophenol, 2-Ethyl-5-di(β-hydroxyethyl)aminophenol, 2-Methoxy-5-di(β-hydroxyethyl)aminophenol, 2-Ethoxy-5-di(β-hydroxyethyl)aminophenol, 2-(β-Hydroxyethyl)-5-di(β-hydroxyethyl)aminophenol, 3-Pyrrolidin-1-ylphenol, 2-Methyl-5-pyrrolidin-1-ylphenol, 2-Ethyl-5-pyrrolidin-1-ylphenol, 2-Methoxy-5-pyrrolidin-1-ylphenol, 2-Ethoxy-5-pyrrolidin-1-ylphenol, 2-(β-Hydroxyethyl)-5-pyrrolidin-1-ylphenol, 3-Piperidin-1-ylphenol, 2-Methyl-5-piperidin-1-ylphenol, 2-Ethyl-5-piperidin-1-ylphenol, 2-Methoxy-5-piperidin-1-ylphenol, 2-Ethoxy-5-piperidin-1-ylphenol, 2-(β-Hydroxyethyl)-5-piperidin-1-ylphenol, 3-Piperazin-1-ylphenol, 2-Methyl-5-piperazin-l-ylphenol, 2-Ethyl-5-piperazin-1-ylphenol, 2-Methoxy-5-piperazin-1-ylphenol, 2-Ethoxy-5-piperazin-1-ylphenol, 2-(β-Hydroxyethyl)-5-piperazin-1-ylphenol, 3-(4-Methylpiperazin-1-yl)phenol, 2-Methyl-5-(4-methylpiperazin-1-yl)phenol, 2-Ethyl-5-(4-methyl-piperazin-1-yl)phenol, 2-Methoxy-5-(4-methyl-piperazin-1-yl)phenol, 2-Ethoxy-5-(4-methylpiperazin-1-yl)phenol, 2-(β-Hydroxyethyl)-5-(4-methylpiperazin-1-yl)phenol, 3-(4-Ethylpiperazin-1-yl)phenol, 2-Methyl-5-(4-ethylpiperazin-1-yl)phenol, 2-Ethyl-5-(4-ethylpiperazin-1-yl)-phenol, 2-Methoxy-5-(4-ethylpiperazin-1-yl)phenol, 2-Ethoxy-5-(4-ethylpiperazin-1-yl)phenol, 2-(β-Hydroxyethyl)-5-(4-ethylpiperazin-1-yl)phenol, 3-(4-(β-Hydroxyethyl)piperazin-1-yl)phenol, 2-Methyl-5-(4-(β-hydroxyethyl)piperazin-1-yl)phenol, 2-Ethyl-5-(4-(β-hydroxyethyl)piperazin-1-yl)-phenol, 2-Methoxy-5-(4-(β-hydroxyethyl)piperazin-1-yl)phenol, 2-Ethoxy-5-(4-(β-hydroxyethyl)-piperazin-1-yl)phenol, 2-(β-Hydroxyethyl)-5-(4-(β-hydroxyethyl)piperazin-1-yl)phenol, 3-Morpholin-4-ylphenol, 2-Methyl-5-morpholin-4-ylphenol, 2-Ethyl-5-morpholin-4-ylphenol, 2-Methoxy-5-morpholin-4-ylphenol, 2-Ethoxy-5-morpholin-4-ylphenol und 2-(β-Hydroxyethyl)-5-morpholin-4-ylphenol ausgewählt sind.

6. Zusammensetzung nach Anspruch 5, in der das bzw. die substituierten meta-Aminophenole der Formel (II) aus 5-N-(β-Hydroxyethylamino)-2-methylphenol, 5-Amino-2-methylphenol und 6-Chlor-2-methyl-5-aminophenol ausgewählt sind.

7. Zusammensetzung nach einem der vorhergehenden Ansprüche, in der das Molverhältnis von erstem Kuppler zu erster Oxidationsbase zwischen 2 und 5 liegt.

8. Zusammensetzung nach einem der vorhergehenden Ansprüche, in der das Molverhältnis von erster Oxidationsbase zu erster Oxidationsbase zwischen 0,7 und 1,3 liegt.

9. Zusammensetzung nach einem der vorhergehenden Ansprüche, die mindestens eine weitere Oxidationsbase umfasst, die aus para-Aminophenolen ausgewählt ist.

10. Zusammensetzung nach Anspruch 9, die mindestens eine zusätzliche Oxidationsbase umfasst, die aus para-Aminophenol und Additionssalzen davon ausgewählt ist.

11. Zusammensetzung nach einem der vorhergehenden Ansprüche, die mindestens einen zusätzlichen Kuppler umfasst, der aus 2-Amino-3-hydroxypyridin und 1,3-Dihydroxy-2-methylbenzol und Additionssalzen davon ausgewählt ist.

12. Zusammensetzung nach einem der vorhergehenden Ansprüche, die außerdem ein Oxidationsmittel umfasst.

13. Verfahren zum Färben von Keratinfasern, **dadurch gekennzeichnet, dass** man auf die Keratinfasern eine Zusammensetzung gemäß einem der Ansprüche 1 bis 11 in Gegenwart eines Oxidationsmittels über einen zur Entwicklung der gewünschten Färbung ausreichenden Zeitraum aufbringt.

14. Vorrichtung mit mehreren Kompartimenten, in der ein erstes Kompartiment eine Färbezusammensetzung gemäß einem der Ansprüche 1 bis 11 enthält und ein zweites Kompartiment ein Oxidationsmittel enthält.

15. Verwendung einer Zusammensetzung gemäß einem der Ansprüche 1 bis 12 zum Oxidationsfärben von Keratinfasern.

16. Verwendung nach Anspruch 15 zum Erhalt einer Färbung auf natürlichen oder dauergewellten grauen Haaren mit 90% weißen Haaren mit einem L*-Wert kleiner oder gleich 50, einem a*-Wert zwischen 10 und 25, einem b*-Wert zwischen 3 und 25 und einem b*/a*-Verhältnis zwischen 0,3 und 1 gemäß der CIELab-Notation.

## Claims

1. Composition for dyeing keratin fibres, comprising, in a suitable medium:
• at least one first oxidation base chosen from 2,3-diamino-6,7-dihydro-1H,5H-pyrazolo[1,2-a]pyrazol-1-one, and the addition salts thereof;
• at least one second oxidation base chosen from para-phenylenediamine and para-tolylenediamine, and the addition salts thereof; and
• at least one first coupler chosen from the substituted meta-aminophenols of formula (II) below, and the addition salts thereof: 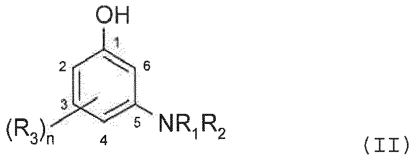 in which:
R₁ and R₂, which may be identical or different, represent a hydrogen atom; an alkyl radical; a monohydroxyalkyl radical; a polyhydroxyalkyl radical; a monoaminoalkyl radical; or
R₁ and R₂ form, together with the nitrogen atom to which they are attached, a saturated or unsaturated, 5- to 7-membered cyclic group containing one or more heteroatoms, which may be unsubstituted or substituted with one or more radicals chosen from carboxyl, carboxamido, hydroxyl, amino, monoalkylamino and dialkylamino radicals, and alkyl radicals optionally substituted with one or more hydroxyl, amino, monoalkylamino or dialkylamino radicals;
R₃ represent, independently of each other, a halogen atom; an alkyl radical; an alkoxy radical; a monohydroxyalkyl radical; a polyhydroxyalkyl radical; a monohydroxyalkoxy radical; a polyhydroxyalkoxy radical;
n is an integer between 0 and 4;
with the proviso that when n is equal to 0, then at least one of the radicals R₁ and R₂ is other than a hydrogen atom;
it being understood that the first coupler/first oxidation base mole ratio is greater than 1, the first oxidation base/second oxidation base mole ratio is between 0.5 and 1.5 and the molar amount of the first oxidation base is greater than or equal to 2.5×10⁻³ mol per 100 g of composition.

2. Composition according to Claim 1, in which R₁ and R₂ represent, independently of each other, a hydrogen atom; an alkyl radical; a monohydroxyalkyl radical; or R₁ and R₂ form, together with the nitrogen atom to which they are attached, a ring chosen from pyrrolidine, piperidine, homopiperidine, piperazine, homopiperazine and morpholine heterocycles; the said rings possibly being substituted with one or more hydroxyl, amino, mono(C₁-C₂)alkylamino, di(C₁-C₂)alkylamino, carboxyl or carboxamido radicals, or C₁-C₄ alkyl radicals optionally substituted with one or more hydroxyl, amino, mono(C₁-C₂)alkylamino or di(C₁-C₂)alkylamino radicals.

3. Composition according to Claim 1 or 2, in which R₃ is chosen from a halogen atom, an alkyl radical, an alkoxy radical and a monohydroxyalkoxy radical.

4. Composition according to any one of the preceding claims, in which n is between 0 and 2.

5. Composition according to any one of the preceding claims, in which the substituted meta-aminophenol(s) of formula (II) is (are) chosen from 5-amino-2-methoxyphenol, 5-amino-2-(β-hydroxyethyloxy)phenol, 5-amino-2-methylphenol, 5-N-(β-hydroxyethyl)amino-2-methylphenol, 5-N-(β-hydroxyethyl)amino-4-methoxy-2-methylphenol, 5-amino-4-methoxy-2-methylphenol, 5-amino-4-chloro-2-methylphenol, 6-chloro-2-methyl-5-aminophenol, 5-amino-2,4-dimethoxyphenol, 5-(γ-hydroxy-propylamino)-2-methylphenol, 3-dimethylaminophenol; 2-methyl-5-dimethylaminophenol; 2-ethyl-5-dimethylaminophenol; 2-methoxy-5-dimethylaminophenol; 2-ethoxy-5-dimethylaminophenol; 2-(β-hydroxyethyl)-5-dimethylaminophenol; 3-diethylaminophenol; 2-methyl-5-diethylaminophenol; 2-ethyl-5-diethylaminophenol; 2-methoxy-5-diethylaminophenol; 2-ethoxy-5-diethylaminophenol; 2-(β-hydroxyethyl)-5-diethylaminophenol; 3-di(β-hydroxy-ethyl)aminophenol; 2-methyl-5-di(β-hydroxyethyl)amino-phenol; 2-ethyl-5-di(β-hydroxyethyl)aminophenol; 2-methoxy-5-di(β-hydroxyethyl)aminophenol; 2-ethoxy-5-di(β-hydroxyethyl)aminophenol; 2-(β-hydroxyethyl)-5-di(β-hydroxyethyl)aminophenol; 3-pyrrolidin-1-ylphenol; 2-methyl-5-pyrrolidin-1-ylphenol; 2-ethyl-5-pyrrolidin-1-ylphenol; 2-methoxy-5-pyrrolidin-1-ylphenol; 2-ethoxy-5-pyrrolidin-1-ylphenol; 2-(β-hydroxyethyl)-5-pyrrolidin-1-ylphenol; 3-piperidin-1-ylphenol; 2-methyl-5-piperidin-1-ylphenol; 2-ethyl-5-piperidin-1-ylphenol; 2-methoxy-5-piperidin-1-ylphenol; 2-ethoxy-5-piperidin-1-ylphenol; 2-(β-hydroxyethyl)-5-piperidin-1-ylphenol; 3-piperazin-1-ylphenol; 2-methyl-5-piperazin-1-ylphenol; 2-ethyl-5-piperazin-1-ylphenol; 2-methoxy-5-piperazin-1-ylphenol; 2-ethoxy-5-piperazin-1-ylphenol; 2-(β-hydroxyethyl)-5-piperazin-1-ylphenol; 3-(4-methylpiperazin-1-yl)phenol; 2-methyl-5-(4-methyl-piperazin-1-yl)phenol; 2-ethyl-5-(4-methylpiperazin-1-yl)phenol; 2-methoxy-5-(4-methylpiperazin-1-yl)phenol; 2-ethoxy-5-(4-methylpiperazin-1-yl)phenol; 2-(β-hydroxyethyl)-5-(4-methylpiperazin-1-yl)phenol; 3-(4-ethylpiperazin-1-yl)phenol; 2-methyl-5-(4-ethylpiper-azin-1-yl)phenol; 2-ethyl-5-(4-ethylpiperazin-1-yl)-phenol; 2-methoxy-5-(4-ethylpiperazin-1-yl)phenol; 2-ethoxy-5-(4-ethylpiperazin-1-yl)phenol; 2-(β-hydroxyethyl)-5-(4-ethylpiperazin-1-yl)phenol; 3-(4-(β-hydroxyethyl)piperazin-1-yl)phenol; 2-methyl-5-(4-(β-hydroxyethyl)piperazin-1-yl)phenol; 2-ethyl-5-(4-(β-hydroxyethyl)piperazin-1-yl)phenol; 2-methoxy-5-(4-(β-hydroxyethyl)piperazin-1-yl)phenol; 2-ethoxy-5-(4-(β-hydroxyethyl)piperazin-1-yl)phenol; 2-(β-hydroxyethyl)-5-(4-(β-hydroxyethyl)piperazin-1-yl)phenol; 3-morpholin-4-ylphenol; 2-methyl-5-morpholin-4-ylphenol; 2-ethyl-5-morpholin-4-ylphenol; 2-methoxy-5-morpholin-4-ylphenol; 2-ethoxy-5-morpholin-4-ylphenol; 2-(β-hydroxyethyl)-5-morpholin-4-ylphenol.

6. Composition according to Claim 5, in which the substituted meta-aminophenol(s) of formula (II) is (are) chosen from 5-N-(β-hydroxyethylamino)-2-methylphenol, 5-amino-2-methylphenol and 6-chloro-2-methyl-5-aminophenol.

7. Composition according to any one of the preceding claims, in which the first coupler/first oxidation base mole ratio is between 2 and 5.

8. Composition according to any one of the preceding claims, in which the first oxidation base/second oxidation base mole ratio is between 0.7 and 1.3.

9. Composition according to any one of the preceding claims, comprising at least one additional oxidation base chosen from para-aminophenols.

10. Composition according to Claim 9, comprising at least one additional oxidation base chosen from para-aminophenol, and the addition salts thereof.

11. Composition according to any one of the preceding claims, comprising at least one additional coupler chosen from 2-amino-3-hydroxypyridine and 1,3-dihydroxy-2-methylbenzene, and the addition salts thereof.

12. Composition according to any one of the preceding claims, also comprising an oxidizing agent.

13. Process for dyeing keratin fibres, **characterized in that** a composition as defined in any one of Claims 1 to 11 is applied to the keratin fibres in the presence of an oxidizing agent, for a time that is sufficient to develop the desired coloration.

14. Multi-compartment device, in which a first compartment contains a dye composition as defined in any one of Claims 1 to 11 and a second compartment contains an oxidizing agent.

15. Use, for the oxidation dyeing of keratin fibres, of a composition as defined in any one of Claims 1 to 12.

16. Use according to Claim 15, for obtaining a coloration on natural or permanent-waved grey hair containing 90% white hairs having, according to the CIELab notation, a value for L* of less than or equal to 50, a value for a* of between 10 and 25, a value for b* of between 3 and 25 and a ratio b*/a* of between 0.3 and 1.
